Europäisches Patentamt

European Patent Office

Office européen des brevets

(19)

(11) Numéro de publication : **0 462 187 B1**

# FASCICULE DE BREVET EUROPEEN

(12)

(45) Date de publication du fascicule du brevet :
21.07.93 Bulletin 93/29

(21) Numéro de dépôt : **90904636.9**

(22) Date de dépôt : **06.03.90**

(86) Numéro de dépôt international :
**PCT/FR90/00151**

(87) Numéro de publication internationale :
**WO 90/10459 20.09.90 Gazette 90/22**

(51) Int. Cl.$^5$ : **A61K 39/12, A61K 39/285,
// C12N15/37, C12N15/86**

(54) **COMPOSITION PHARMACEUTIQUE, UTILE A TITRE PREVENTIF OU CURATIF CONTRE LES TUMEURS INDUITES PAR LES PAPILLOMAVIRUS.**

(30) Priorité : **06.03.89 FR 8902897**

(43) Date de publication de la demande :
**27.12.91 Bulletin 91/52**

(45) Mention de la délivrance du brevet :
**21.07.93 Bulletin 93/29**

(84) Etats contractants désignés :
**AT BE CH DE DK ES FR GB IT LI LU NL SE**

(56) Documents cités :
**The Journal of General Virology ,vol.69, no.6, June 1988 (SGM,GB) H.M. Browne et al.: "Analysis of the L1 gene product of human papillomavirus type 16 by expression in a vaccinia virus recombinant".pages 1263-1273
The Embo Journal, vol. 7, no. 6, juin 1988, IRL Press Ltd (Oxford, GB) A. Storey et al.: "Comparison of the in vitro transforming activities of human papillomavirus types" pages 1815-1820
Nature, vol. 326, 30 avril 1987, R. Lathe et al.: "Tumour prevention and rejection with recombinant vaccinia", pages 878-880**

(56) Documents cités :
**Journal of Cellular Biochemistry, Suppl. 13C, 1989, UCLA Symposia on Molecular & Cellular Biology Symposium Papillomaviruses held from March, 11-18, 1989 G. Meneguzzi et al.: "Vaccination against papillomavirus-induced tumors using Vaccinia recombinants expressing non-structural proteins" voir page 210, résumé 1223**

(73) Titulaire : **TRANSGENE S.A.
11, rue de Molsheim
F-67000 STRASBOURG (FR)**

(72) Inventeur : **MENEGUZZI, Guerrino
Villa Bagatelle Avenue Ste-Colette
F-06100 Nice (FR)**
Inventeur : **LATHE, Richard
1A, The Avenue
GB-Leeds LS8 (GB)**
Inventeur : **KIENY Marie-Paule
7, rue Aloise Quintenz
F-67000 Strasbourg (FR)**

(74) Mandataire : **Martin, Jean-Jacques et al
Cabinet REGIMBEAU 26, Avenue Kléber
F-75116 Paris (FR)**

Jouve, 18, rue Saint-Denis, 75001 PARIS

## Description

La présente invention concerne de nouvelles compositions vaccinales utiles contre les tumeurs induites par les papillomavirus, et plus particulièrement contre les types HPV-16, HPV-18, HPV-31, HPV-33, HPV-35,

Les papillomavirus représentent un groupe de virus à ADN. Ils possèdent une coque protéique et un génome d'ADN circulaire d'environ 7900 paires de bases. Un certain nombre de types de papillomavirus, bovins (BPV), humains (HPV) ont été identifiés, et les génomes de certains ont été complètement séquencés (1).

Les travaux de recherche fondamentale qui ont été menés sur ces virus ont ainsi conduit à distinguer dans leur génome une région précoce et une région tardive, par analogie avec le génome du virus du polyome et du SV40. La région tardive contient deux cadres de lecture L1 et L2 qui codent pour les composants majeurs de la capside. La région précoce contient au moins les cadres de lecture E1, E2, E4, E5, E6, E7 et E8. Les protéines codées par ces cadres de lecture possèdent des fonctions différentes. Trois de ces protéines sont impliquées dans les processus de transformation oncogénique des cellules infectées. La protéine E5 de BPV-1 qui possède un important pouvoir de transformation et qui peut in vitro transformer des cellules de façon indépendante (2) est codée par la partie 3' de la région précoce. Les protéines E6 de BPV-1 et E7 de HPV-16 sont codées par la partie 5' de la région précoce et sont impliquées dans l'induction et le maintien de la transformation oncogène. Ces deux protéines semblent dériver d'un gène ancestral commun par duplications successives d'un peptide de 33 acides aminés (3). Le pouvoir transformant de E7 a été démontré pour HPV-16 et 18 (4, 5, 6). Parmi les autres protéines précoces, E1 et E2 possèdent un rôle dans la réplication et/ou l'expression du virus alors qu'il n'a pas été mis en évidence de fonction à E4 et E8.

Chez l'homme, les HPV sont associés à des pathologies allant de l'infection bénigne de la peau, aux verrues et aux tumeurs malignes. Ces virus sont hautement spécifiques des tissus cibles, notamment les épithéliums de l'épiderme des voies génitales, orales et respiratoires (7). Les données épidémiologiques suggèrent fortement le rôle de certaines souches d'HPV dans le cancer du col de l'utérus et des voies basses (tumeur le plus fréquemment fatal chez la femme). L'ADN de HPV-16 et HPV-18 est retrouvé dans la plupart des biopsies provenant de cellules cancéreuses génitales, plus rarement on détecte HPV-31, HPV-33, HPV-35, HPV-39 et HPV-45.

Les pathologies associées aux virus HPV posent un problème thérapeutique du fait de leur nature persistante et récurrente. De nombreuses approches ont déjà été utilisées dans le traitement de ces maladies : la chirurgie, la chimiothérapie, les agents antiviraux et l'immunothérapie (8).

En particulier, la publication européenne de brevet EP-A-0133123 décrit une approche de vaccination contre une infection par les papillomavirus qui consiste à préparer par génie génétique des protéines de la capside du virus, c'est-à-dire des protéines de structure correspondant donc à la région tardive du virus, et à les utiliser comme agents immunogènes. Dans ce document, les moyens décrits visent à une protection contre une infection par les virus eux-mêmes, et donc à priori contre toutes les formes d'infections susceptibles de se développer.

La présente invention vise plus précisément l'obtention de vaccins actifs, à titre préventif ou curatif contre les tumeurs malignes résultant d'une infection préétablie par HPV.

La présente invention repose sur l'observation que la formation des tumeurs induites par les papillomavirus est due à l'expression des gènes précoces des virus.

L'invention a donc pour objet des compositions pharmaceutiques caractérisées en ce qu'elles contiennent à titre de principe actif préventif ou curatif au moins un poxvirus recombinant qui comporte une séquence d'ADN hétérologue codant au moins pour la région essentielle d'une protéine précoce d'un papillomavirus ainsi que les éléments de régulation assurant son expression dans les cellules supérieures.

Par région essentielle de ladite protéine, on entend désigner la partie de la séquence protéique capable d'induire une immunité antitumorale.

Les compositions vaccinales selon l'invention peuvent être utilisées à des fins variées. Elles peuvent être utilisées à titre préventif, pour empêcher l'apparition de tumeurs malignes, soit avant toute infection par le virus, soit encore après une infection ayant causé des troubles bénins, par crainte de voir d'autres tissus atteints et se transformer en tissus cancéreux. Elles peuvent potentiellement être aussi utilisées à titre curatif, pour faire disparaître une tumeur déjà installée, ou en complément ou substitution à une ablation. Ces utilisations peuvent concerner l'homme, mais aussi l'animal, en particulier les bovins pour la prévention des infections par le virus BPV. On pourra utiliser, le cas échéant le poxvirus recombinant avec un support pharmaceutiquement acceptable pour son inoculation à l'homme ou à l'animal.

Etant données les observations qui ont été faites, et rappelées plus haut sur la fréquence d'infection par HPV de type 16-18-31-33-35-39 et 45 dans les cas de cancer du col de l'utérus, l'invention concerne plus particulièrement des compositions vaccinales utiles contre HPV-16, HPV-18, HPV31, HPV33, HPV-35, HPV-39 et HPV-45.

C'est pourquoi l'invention a pour objet une composition vaccinale telle que définie précédemment dans laquelle le poxvirus comprend au moins une séquence d'ADN codant pour au moins une protéine non-structurale du virus HPV de type 16, 18, 31, 33, 35, 39 ou 45 et en particulier du virus HPV-16.

Comme rappelé précédemment, la structure des virus de la famille des papillomes, en particulier BPV-I et HVP, est telle qu'il existe au moins 7 cadres de lectures susceptibles de correspondre à des fonctions protéiques bien spécifiques des mécanismes d'action du virus et du maintien du génome viral. C'est pourquoi il peut être intéressant de préparer des compositions pharmaceutiques qui expriment dans l'organisme humain plusieurs protéines simultanément. Ceci peut être obtenu soit avec plusieurs poxvirus exprimant chacun une protéine donnée, soit avec un poxvirus contenant plusieurs séquences d'ADN hétérologue correspondant aux protéines choisies.

Bien entendu, le poxvirus recombinant comportera l'ensemble des éléments nécessaires à l'expression des protéines dans les cellules supérieures, c.à.d. généralement un promoteur de la transcription du gène et une région d'initiation de la traduction dans la cellule hôte ; de préférence le promoteur sera le promoteur d'un gène du poxvirus utilisé.

Parmi tous les poxvirus envisageables, on choisit de préférence le virus de la vaccine, car il a déjà été établi que les antigènes exprimés par le virus de la vaccine recombinant sont correctement présentés à la surface des cellules infectées et permettent l'induction d'une réponse immunitaire de type cellulaire. Ces circonstances sont donc particulièrement favorables puisqu'il est connu que l'élimination des cellules tumorales implique l'immunité cellulaire.

Lorsque le poxvirus est le virus de la vaccine, on utilisera de préférence comme promoteur celui du gène de la protéine 7,5 K du virus de la vaccine. L'ensemble promoteur-séquence codante sera inséré dans une région non essentielle du virus ; par exemple, dans le cas du virus de la vaccine, le gène de la thymidine kinase (TK) ce qui permettra une sélection aisée des virus recombinants TK⁻.

Généralement, le virus vivant est inoculé à l'homme ou à l'animal. Toutefois, on peut aussi envisager d'injecter à l'homme ou à l'animal le virus recombinant tué, présentant à sa surface les protéines choisies, ou les protéines purifiées à partir de cultures cellulaires infectées par les virus de la vaccine recombinants.

Les compositions pharmaceutiques selon l'invention peuvent être préparées selon des procédés connus dans le domaine des vaccins, et les doses applicables pourront varier dans une large gamme. Elles seront fonction notamment de l'état du patient et d'autres paramètres qui seront évalués par le praticien.

L'invention sera illustrée par les exemples ci-après, qui décrivent d'une part des résultats de vaccination sur animaux auxquels on a injecté des cellules transformées par le virus du papillome bovin (BPV-1) et, d'autre part, des résultats de vaccination sur animaux auxquels on a injecté des cellules transformées par le virus du papillome humain (HPV-16). On décrit en particulier le clonage et le séquençage des ADN correspondant aux cadres de lecture aux protéines E5, E6, E7 de HPV-16 ainsi que les résultats de vaccination obtenus avec des virus recombinants exprimant ces protéines.

La description détaillée de l'invention sera accompagnée par les figures 1 à 7 qui représentent :
- la figure 1 représente le gel SDS-PAGE des immunoprécipités des produits des cadres de lecture E1, E2, E5 et E6 de la région précoce du génome du papillomavirus bovin BPV-1.
- la figure 2 représente la période de latence du développement des tumeurs des animaux vaccinés avec les virus de la vaccine recombinants, exprimant les produits des cadres de lecture E1, E2, E5, E6 ou E7 du papillomavirus bovin BPV-1 (VVbE1, VVbE2, VVbE5, VVbE6 et VVbE7), ou un virus de la vaccine recombinant témoin, VV-0 (n'exprimant pas une protéine de BPV-1) et éprouvés avec des cellules FR3T3 transformées par BPV-1 (FR3T3 BPV-1-6).
- la figure 3 représente la période de latence du développement des tumeurs chez des animaux vaccinés avec les virus de la vaccine recombinants exprimant les produits des cadres de lecture E5, E6 et E7 du papillomavirus bovin BPV-1 (VVbE5, VVbE6, VVbE7) ou une association VVbE5-VVbE6 ou VVbE5-VVbE7 ou un virus de la vaccine recombinant témoin VV-0 (n'exprimant pas une protéine de BPV-1) et éprouvés avec des cellules FR3T3 transformées par BPV-1 (FR3T3-BPV-1-3).
- la figure 4 représente schématiquement la structure du bactériophage M13 E7/E6 (HPV-16).
- la figure 5 représente schématiquement la structure du bactériophage M13 E5 (HPV-16).
- la figure 6 représente la séquence de l'ADN complémentaire du cadre de lecture E5 (HPV-16).
- la figure 7 représente le gel SDS-PAGE des immunoprécipités des produits des cadres de lecture E6 et E7 de la région précoce du génome du papillomarivus humain HPV-16.
- la figure 8 représente l'évolution de la taille des tumeurs chez des animaux vaccinés avec le virus de la vaccine recombinant exprimant le produit du cadre de lecture E6 du papillomavirus humain HPV-16 (VVhE6) et éprouvés avec des cellules primaires de rat transformées par HPV-16 et un oncogène ras.
- la figure 9 représente l'évolution de la taille des tumeurs chez des animaux vaccinés avec le virus de la vaccine recombinant exprimant le produit du cadre de lecture E7 du papillomavirus humain HPV-16

EP 0 462 187 B1

(VVhE7) et éprouvés avec des cellules primaires de rat transformées par HPV-16 et un oncogène ras.

Exemple 1 : Inhibition du développement des tumeurs induites par le virus du papillome bovin (BPV-1) par vaccination par des virus de la vaccine recombinants exprimant les protéines précoces de BPV-1.

a) Construction des virus de la vaccine exprimant les protéines précoces de BPV-1

Le plasmide pM69 (9) contient la région précoce du génome de BPV-1 inséré en tant que fragment HindIII-BamHI dans les sites HindIII et BamHI du plasmide pML2 (9). Des sous-fragments contenant les cadres de lecture E1, E2, E5, E6 et E7 sont excisés par digestion avec des enzymes de restriction (voir tableau I) et introduits dans les bactériophages M13TG130 ou M13TG131 (10) puis soumis à une mutagénèse localisée dirigée par oligonucléotide avant d'être transférés dans le plasmide pTG186 poly (11). Ces étapes sont récapitulées dans le tableau I.

Légende du tableau I :

[a] : les nucléotides soulignés précisent les bases non apariées à la séquence parentale de BPV-1

[b] : le codon d'initiation de la traduction et le site de restriction utilisés dans le clonage sont soulignés.

[c] : il n'a pas été possible d'introduire le fragment EcoRI - BamHI du plasmide pM69 contenant le cadre de lecture E5 dans le bactériophage M13TG131 dans l'orientation désirée : les clones sont obtenus dans l'orientation inversée dans laquelle deux fragments de plasmides indépendants sont clonés ; la mutagénèse par oligonucléotide correspond donc à l'autre brin de l'ADN.

[k] : extrémité produite par digestion par une enzyme de restriction puis traitement avec le fragment Klenow de l'ADN polymérase I de E. coli avant le clonage ;

na : non appliquable.

4

Tableau I : Clonage et mutagénèse des sous-fragments du génome de BPV-1 pour leur transfert dans les virus de la vaccine.

| BPV-1 • cadre de lecture • fragment | • vecteur • site | Mutagénèse • séquence (5'-3') des oligonucléo-tides utilisés [a] • séquences au site d'initiation de la traduction [b] | Clonage • fragment • vecteur • site |
|---|---|---|---|
| E1 NruI-AvrII | M13TG130 BamHI[k]-XbaI | na (TCGCGAGCGTCATGG) | BamHI-SstI pTG186poly BamHI-SstI |
| E2 EcoRI-SpeI | M13TG131 EcoRI-XbaI | GAGGAGGATCCTGAAGAGGA (GGATCCTGAAGAGGATGG) | BamHI pTG186poly BamHI |
| E5 EcoRI-BamHI | M13TG131 EcoRI-BamHI | AGATTTGCCATAGTCGACCAGTCA[c] (GTCGACTATGG) | SalI-BamHI pTG186poly SalI-BamHI |
| E6 HpaI-SmaI | M13TG130 SmaI | CAGACCCCGGATCCAACATGGACCT (GGATCCAACATGG) | BamHI-XmaIII[k] pTG186poly BamHI-SmaI |
| E7 HpaI-SmaI | M13TG130 SmaI | TGCTACGACTCGAGCAAACATGGTTCA (CTCGAGCAAACATGG) | XhoI-EcoRI pTG186poly, SalI-EcoRI |

La mutagénèse localisée dirigée par oligonucléotide permet d'introduire une séquence consensus de l'initiation de la traduction au niveau du codon d'initiation de manière à assurer l'expression correcte après transfert dans le virus de la vaccine. Dans chaque cas, l'oligonucléotide de synthèse introduit un site de restriction unique juste avant la séquence déterminant le début de la traduction.

Dans le cas du cadre de lecture de E1, il n'est pas nécessaire de faire de mutagénèse localisée du fait de la présence d'un site de restriction dans le génome de BPV-1 (site NruI en position -11 par rapport à l'ATG initiateur et d'une séquence quasi consensus de la région d'initiation de la traduction (GCGTCATGG) : le nucléotide G en position -3 est presque aussi efficace que A dans l'initiation de la traduction.

Des cellules primaires d'embryon de poulet sont cultivées à 37°C dans un milieu MEM (Gibco) supplémenté avec 10 % de sérum de veau foetal. Elles sont simulanément soumises à une infection par un virus de la vaccine thermosensible et à une transfection par les vecteurs d'expression/transfert portant les segments insérés de BPV-1 et par l'ADN du virus de la vaccine type sauvage. Après sélection, on isole selon les techniques courantes, des recombinants de la vaccine dans lesquels l'expression de la séquence insérée est sous le contrôle du promoteur 7.5 K du virus de la vaccine.

Les virus de la vaccine recombinants (VV) exprimant les protéines précoces de BPV-1 E1, E2, E5, E6 et

5

E7 sont appelés respectivement VVbE1, VVbE2, VVbE5, VVbE6 et VVbE7.

b) Expression de E1, E2, E5, E6 et E7 dans les cellules infectées par les virus recombinants

Des cellules BHK-21 sur milieu de Eagle modifié par Dulbecco MEM.BME (GIBCO) supplémenté avec 10 % de sérum de veau foetal (GIBCO) sont infectées avec l'un des six virus recombinants avec une multiplicité d'approximativement 20 unités formant plaque (ufp) par cellule.

Après une heure à 37°C, du milieu frais est ajouté et les cellules sont incubées pendant deux heures. Le milieu est retiré, les cellules lavées une fois. Du milieu MEM.BME sans méthionine et/ou cystéine et supplémenté avec 5 % de sérum de veau foetal dialysé est alors ajouté. Le marquage est effectué avec 0.5-1 mCi/ml de L-méthionine-35S et/ou L-cystéine-35S (Amersham) pendant 3 heures à 37°C. Les cellules ainsi marquées sont rincées deux fois avec 20 mM Tris. HCl pH 7,2 ; 150 mM NaCl (tampon TS) contenant de l'aprotinine (1 UI/ml ; Biosys, France), collectées par grattage et rincées par 2 centrifugations dans du tampon STE (20 mM Tris. HCl pH 7,2 ; 150 mM NaCl ; 1 mM $Na_2$EDTA ; 1 % aprotinine). Les cellules sont lysées dans du tampon RIPA [50 mM Tris. HCl ; pH 7,4 ; 150 mM NaCl ; 1 mM $NA_2$EDTA ; 1 % Triton X-100 ; 1 % NaDeoxycholate ; 0,1 % SDS] ou fractionnées pour déterminer la localisation subcellulaire des protéines de BPV-1.

L'immunoprécipitation et le SDS-PAGE sont effectués suivant la procédure décrite par Davis (12). Des antisera polyclonaux de lapins, dressés contre les protéines de fusion bactériennes correspondant aux cadres de lecture E1, E2, E5, E6 et E7 sont obtenus par la méthode classique connue de l'homme du métier, voir par exemple les références (13) et (14).

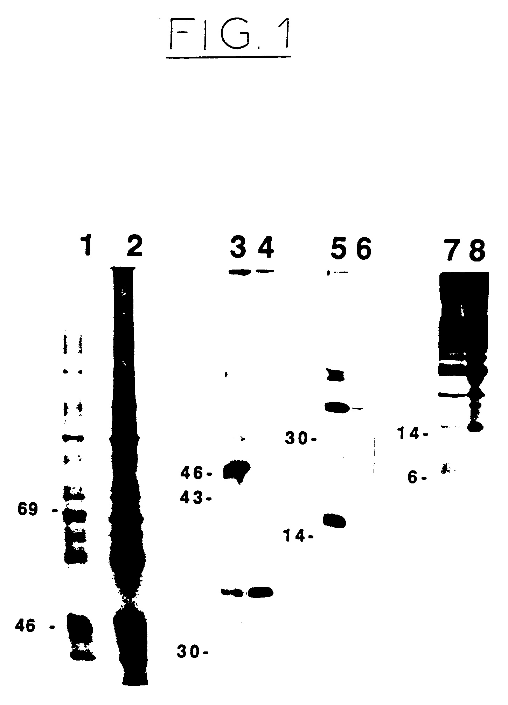

Les cellules infectées avec VVbE1 produisent une protéine nucléaire de 69kD qui est spécifiquement reconnue par l'antisérum anti-E1 (fig. 1, bande 1, la bande 2 correspond à une immunprécipitation avec un sérum non immun). Les cellules infectées par VVbE2 contiennent un niveau élevé d'un polypeptide de 48 kD présentant une réactivité croisée avec E2 et correspondant à la protéine de transactivation majeure codée par le cadre de lecture E2 de BPV-1 (fig. 1, bande 3 et contrôle, bande 4). Ce polypeptide est détecté dans toutes les sous-fractions cellulaires examinées, conformément à ce qui a été décrit pour la protéine E2 de cellules transformées par BPV-1 (15). VVbE6 code pour une protéine de 15,5 kD spécifiquement précipitée par le sérum anti E6 (fig. 1, bande 6 et contrôle bande 7) et localisée à plus de 50 % dans la fraction cytoplasmique comme pour la protéine E6 des cellules transformées par BPV-1 (16). Les cellules infectées par VVbE5 produisent un polypeptide de 7,5 kD associé aux membranes cellulaires (fig. 1, bande 7 et contrôle, bande 8), localisation caractéristique de la protéine E5 de BPV-1 (17). Les expériences d'immunoprécipitation avec l'antisérum anti-E7 ne présentent pas de réactivité croisée. Néanmoins, l'analyse des vecteurs d'expression révèle la présence du cadre de lecture de E7 dans une orientation correcte et nous supposons que E7 est effectivement produite mais que pour une raison inconnue elle n'est pas précipitée par l'antisérum disponible.

c) Vaccination contre les cellules tumorales induites par BPV-1

Des groupes de rats femelles (Fischer) âgées de 4 semaines sont inoculés par voie intradermale ou intrapéritonale avec les différents virus recombinants (à une dose de $10^8$ ufp dans 100 μl). Ils subissent une injection de rappel avec la même dose après 12 jours et sont soumis à une inoculation d'épreuve, entre le 16e et le 17e jour avec des cellules 3T3 de lignées syngéniques de rats Fischer (FR3T3) transformées par BPV-1 selon le protocole décrit dans les références (9, 18) et appelées FR3T3-BPV-1-6. Pour ceci, $2 \times 10^4$ cellules sont injectées par voie sous-cutanée dans un volume de 200 μl de milieu MEM.BME sans sérum.

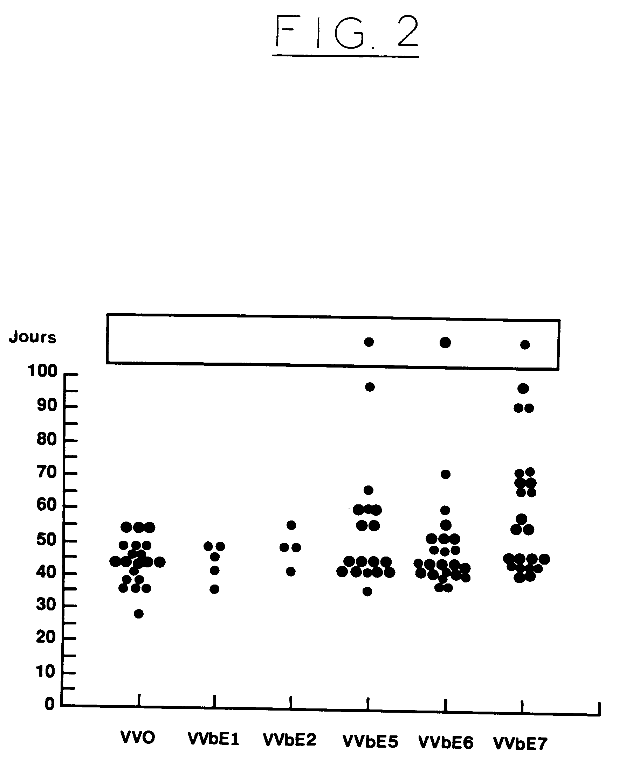

Les résultats sont représentés sur la figure 2 :
- tous les animaux contrôlés, vaccinés avec VV-0 développent des tumeurs après une période de latence de 43 jours en moyenne.
- les animaux vaccinés avec VVbE1 ou VVbE2 ne présentent pas de modification dans le developpement des tumeurs.
- les animaux vaccinés avec VVbE5, VVbE6 et VVbE7 présentent un retard significatif de l'apparition des tumeurs. De plus dans quelques cas, il n'y a pas de développement de tumeurs (> 250 jours après l'épreuve), points représentés dans le cadre en haut de la figure.

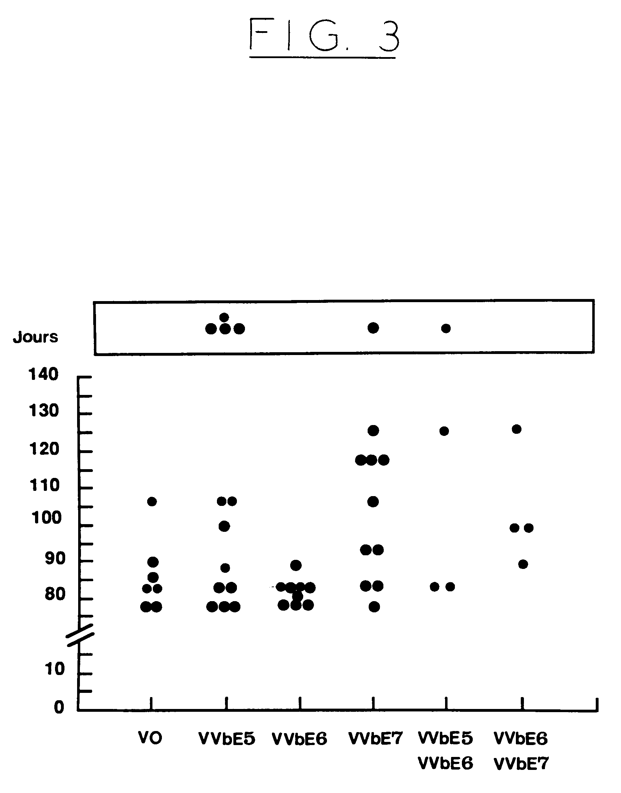

Ces expériences sont répétées en effectuant l'inoculation d'épreuve avec des cellules de la lignée FR3T3 transformées par BPV-1 selon le protocole décrit dans les références (9, 18) et appelées FR3T3-BPV-1-3. VVbE1 et VVbE2 étant sans effet, seuls VVbE5, E6 et E7 sont testés et deux groupes de rats sont inoculés avec 2 virus recombinants simultanément, soit VVbE5 et VVbE7 soit VVbE5 et VVbE6 afin de mettre en évidence un éventuel effet cumulatif de ces antigènes. La figure 3 confirme l'effet bénéfique d'une vaccination par VVbE5 et VVbE7 (les points dans le cadre du haut de la figure correspond aux animaux qui ne développent pas de tumeur) alors que VVbE6 reste sans effet notable dans cette situation. De plus il n'apparaît pas d'effet cumulatif marqué lors de l'association VVbE5, VVbE7.

Exemple 2 : Clonage du génome du virus HPV-16.

Les cellules de la lignée CaSki (ATCC 1550) contiennent le génome du virus HPV-16 intégré dans un chromosome. A partir de 10 fioles de 75 cm³ de cellules CaSki semi-confluences, l'ADN génomique total est purifié de la façon suivante : les cellules sont récupérées par grattage, centrifugées et lavées puis reprises dans 15 ml de tampon TE [Tris 10 mM pH 7,5 ; EDTA 1 mM] + 0,5 % SDS. Après traitement à la protéinase K (7,5 mg/15 ml) à 37°C pendant 16 heures, l'ADN est stocké à 4°C. De cette façon, 1 ml de solution contenant 0,6 mg d'ADN est obtenu.

70 µg de cet ADN sont soumis à une digestion partielle par l'enzyme MboI (10 min. ; 40 unités) puis déposés sur un gradient linéaire (20 % - 40 %) de sucrose. Après centrifugation (rotor SW28, 16 heures à 25.000 t, 20°C), des fractions de 500 µl sont collectées. Les fractions contenant de l'ADN de taille comprise entre 10 et 20 kb (fraction 23-26) sont réunies, dialysées contre du tampon TE, puis précipitées à l'éthanol. De cette façon, 5 µg d'ADN sont obtenus.

Afin de disposer d'un vecteur de clonage pour l'ADN décrit précédemment, l'ADN du bactériophage lambda EMBL 301 (19) est digéré par l'enzyme de restriction BamHI. Après extraction au phénol-chloroforme et précipitation à l'éthanol, l'ADN est resuspendu dans du tampon TE.

L'ADN génomique des cellules CaSki et l'ADN du bactériophage lambda sont ensuite ligués selon un protocole classique (1 µg de vecteur, 2 µg d'ADN génomique). Après empaquetage de l'ADN grâce à une trousse commercialisée par Amersham, un total de 0,75 x 10⁶ clones indépendants est obtenu. Ces bactériophages sont étalés avec des bactéries E. coli Q358 sur 18 boîtes de 14 cm de diamètre et sur milieu LBM afin d'être criblés avec des oligonucléotides synthétiques déduits de la séquence du virus HVP-16 [(EMBL) PA16].

Le criblage de l'ADN des bactériophages recombinants est effectué de façon classique pour l'homme de métier. Les oligonucléotides 1817 (séquence 5' CATGCATGGAGATACACCTACATTG 3' ; cadre de lecture E7 et 1818 (séquence 5' GTGGATAACAGCAGCCTCTGCGTTT 3'; cadre de lecture E5) marqués radioactivement au ³²P, sont mélangés et hybridés à l'ADN phagique (transféré sur des filtres de nitrocellulose) pendant 16 heures à 55°C dans un tampon SSC concentré 6 fois. Les filtres sont ensuite lavés dans les mêmes conditions de stringence, 10 signaux positifs (1-10) ont été obtenus. La zone des boîtes de pétri correspondant à ces signaux est reprise dans 1 ml de tampon LBM. Ces suspensions sont réétalées sur des boîtes de pétri ∅ 10 cm (2-10 µl de suspension par boîte) en double et un criblage secondaire est effectué avec les oligonucléotides 1817 et 1818 séparément. Les signaux correspondant aux phages obtenus à partir des premiers isolements 4 et 5 étant les plus intenses, ces deux clones sont choisis pour la suite des expériences.

Des minicultures de bactéries E. coli Q358 infectées avec le bactériophage, isolé à partir des sous-clones 4-1, 4-2, 5-1 et 5-2 sont réalisées. L'ADN de ces clones est purifié et soumis à digestion par l'enzyme de restriction PstI afin d'analyser les recombinants sélectionnés.

Après migration sur un gel d'agarose 1 %, les ADN ci-dessus sont transférés sur une membrane de nitrocellulose selon la techniqe de Southern. Ces membranes sont ensuite incubées avec les deux oligonucléotides 1817 et 1818 marqués au ³²P par action de la polynucléotide kinase comme précédemment. Après hybridation et lavage de la membrane, cette dernière est soumise à autoradiographie. Deux bandes correspondant à des ADN de tailles 1063 et 1772 pb respectivement sont révélées ainsi, indiquant que les clones 4-1, 4-2, 5-1 et 5-2 possèdent le génome du virus HPV-16.

Exemple 3 : Séquençage des ADN correspondant à E6, E7 et E5

Le bactériophage 5-1 est choisi pour les expériences ultérieures. Afin de disposer de quantités importantes de l'ADN de ce clone une préparation d'ADN à partir de 500 ml de bactérie E. coli Q358 infectées est réalisée selon un protocole classique. 800 µg d'ADN sont ainsi obtenus. Après digestion par l'enzyme PstI, l'ADN est soumis à l'électrophorèse sur gel d'agarose 1 % contenant du bromure d'éthidium et visualisé sur une lampe UV. Les bandes de gel correspondant aux tailles 1063 et 1772 pbs sont découpées et l'ADN est extrait en utilisant une trousse "Geneclean" (Bio101 Inc). Cet ADN est ensuite ligué dans un bactériophage M13TG130 ouvert au site PstI et transformé dans des bactéries compétentes E. coli NM522.

L'ADN des bactériophages M13 recombinant est purifié à partir de minicultures (1,5 ml) et analysé par digestion avec des enzymes de restriction. Un clone contenant la bande HPV-16 1063 pb (M13 E5) et un clone contenant la bande HPV-16 1772 pb (M13 E7/E6) sont sélectionnés.

Afin de limiter le travail de séquençage et comme les ADN recherchés sont ceux correspondant aux cadres de lecture E5, E6 et E7, seules les régions des fragments de restriction PstI correspondant aux protéines E6 et E7 (M13 E7/E6) et E5 (M13 E5) sont séquencées, grâce à des oligonucléotides synthétiques de séquence

5' ATCTAACATATATTC 3' et 5' GTTGTTCCATACAAA 3' pour M13 E7/E6
et
5' GTCTGCCTATTAATAC 3' pour M13 E5.

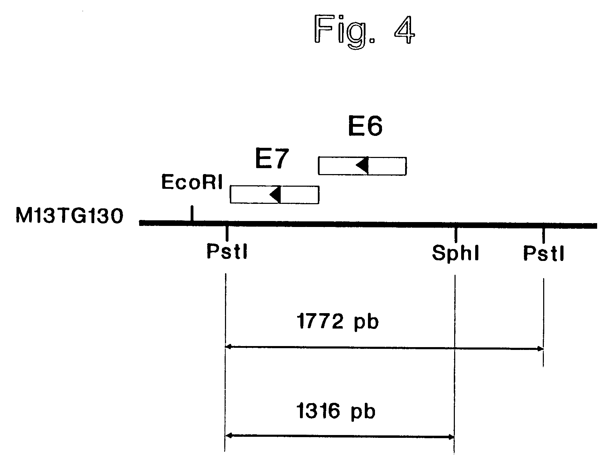

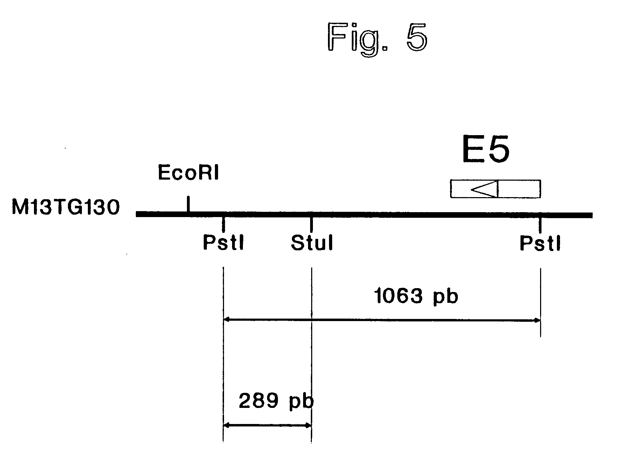

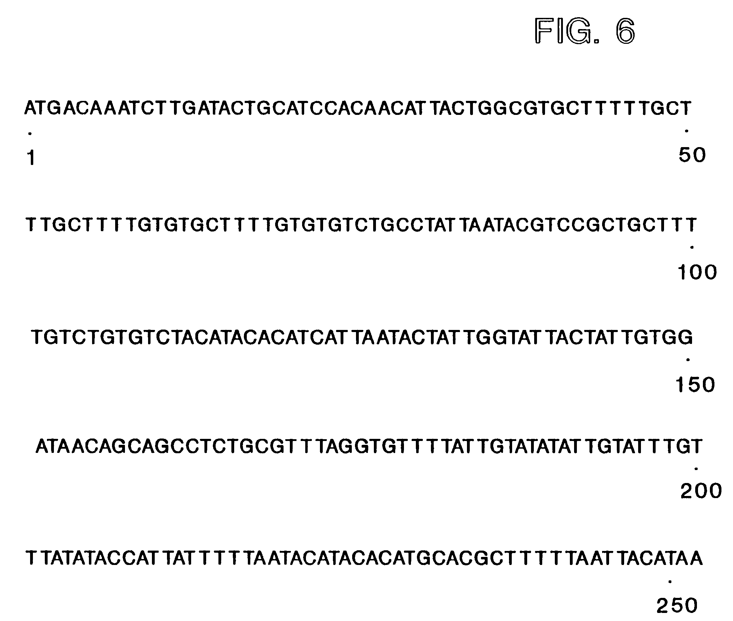

La figure 4 présente la structure du bactériophage M13 E7/E6 et la figure 5 celle du bactériophage M13 E5. La séquence obtenue pour E7 révèle une homologie totale avec la séquence contenue dans les banques de données PPH16. Pour E6 on observe deux mutations : G à la place de A en position +46 et G à la place de T en position +264 par rapport à l'ATG initiateur de E6. Pour E5, on obtient la séquence représentée à la figure 6, qui diffère par quelques bases de la séquence contenue dans la banque de données.

Exemple 4 : Clonage des fragments d'ADN portant E6, E7 et E5 dans le vecteur de transfert.

Avant d'intégrer les ADN codant pour ces 3 cadres de lecture dans des virus de la vaccine recombinants, il est nécessaire de les modifier afin de disposer de sites de restriction uniques en amont et en aval des gènes et d'améliorer les séquences autour de l'ATG initiateur afin d'obtenir une bonne traduction et un niveau élevé de protéine synthétisée.

a) Cadre de lecture E6

Un site de restriction SalI et un site SphI sont créés respectivement en amont et en aval de la séquence codant pour E6 grâce à deux oligonucléotides synthétiques, en employant une technique de mutagénèse localisée dirigée par oligonucléotide (trousse Amersham). Ces deux mutations ponctuelles sont réalisées simultanément en utilisant les oligonucléotides suivants :
5' GTTAGTATAAAAGTCGACCCACCATGCACCAAAAGAG 3'
5' CATGCATGCAGATACACC 3'

Un nucléotide A en position -3 par rapport à l'ATG est introduit à la place d'un nucléotide T en même temps que le site Sal I.

Les bactériophages obtenus sont analysés par digestion avec des enzymes de restriction et par séquençage. Un clone est retenu pour la suite désigné M13TG1181. Le fragment de restriction SalI-SphI est ensuite cloné dans le vecteur pTG186 poly (11) ouvert aux sites SalI et SphI (pTG2198). Ce vecteur permet le transfert de l'ADN dans le génome du virus de la vaccine.

b) Cadre de lecture E7

Un site de restriction PstI et un nucléotide A en position -3 sont introduits en amont de la séquence codant pour la protéine E7 par mutagénèse localisée grâce à l'oligonucléotide suivant :
5' GTAGAGAAACCCTGCAGCCACCATGCATGGAG 3'

Plusieurs clones ont été obtenus. L'un d'entre eux qui contient un fragment de restriction PstI de 350 pb, est séquencé dans la zone correspondant à la mutation. La séquence correspond bien à la mutation attendue (M13TG1182). Le fragment de restriction PstI est ensuite inséré dans le plasmide pTG186 poly ouvert au site PstI (pTG2199).

c) Cadre de lecture E5

Un site de restriction PstI est introduit en amont de l'ATG initiateur du cadre de lecture E5 (position 3851 dans PPH16) par mutagénèse localisée grâce à l'oligonucléotide suivant :
5' GTCTACTGGATTTACTGCAGTATGACAAATCTTGAT 3'

Un site de restriction EcoRI est introduit en aval du codon stop grâce à l'oligonucléotide suivant :
5' GTATATGTACATAATGAATTCTTACATATAATTGTTG 3'

Ces deux mutations sont introduites simultanément (trousse Amersham) . Le clone retenu pour la suite est désigné par M13TG 3151. Le fragment de restriction PstI-EcoRI est ensuite inséré dans le plasmide pTG186 poly ouvert aux sites PstI et EcoRI (pTG3180).

Exemple 5 : Construction des virus de la vaccine recombinants.

Les plasmides pTG2198, 2199 et 3180 sont utilisés pour transférer les cadres de lecture E6, E7 et E5 dans un virus de la vaccine (souche Copenhagen) comme décrit précédemment. Les virus recombinants où les séquences codantes sont sous le contrôle de promoteur 7,5 K de la vaccine sont appelés VVhE6, VVhE7 et VVhE5.

Des cellules BHK-21 sur milieu MEM.BME supplémenté avec 10 % de serum de veau foetal sont infectées avec l'un des trois virus recombinants avec une multiplicité d'approximativement 20 ufp par cellule.

Après incubation suivant le protocole décrit précédemment, les cellules sont récupérées, lysées et fractionnées afin de déterminer la localisation subcellulaire des protéines de HPV-16.

L'expression des gènes E6 et E7 est vérifiée par immunoprécipitation des protéines (marquées à la cystéine $^{35}$S) synthétisées.

Les anticorps polyclonaux dressés contre les protéines de fusion bactériennes correspondant aux cadres de lecture de E6 et E7 sont obtenus par la méthode classique connue de l'homme du métier et décrite dans les références (20) et (21).

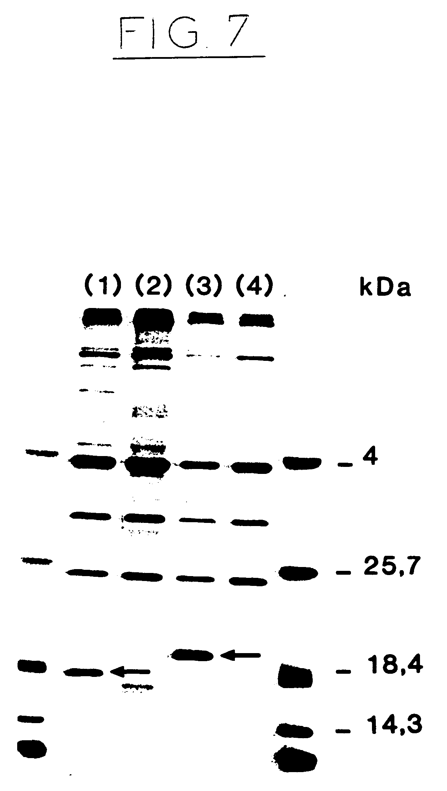

Les cellules infectées par VVhE6 produisent une protéine cytoplasmique de 18 kD qui est spécifiquement reconnue par l'antisérum anti-E6 (fig. 7, bande 1, la bande 2 correspond à une immunoprécipitation avec un sérum non immun). Les cellules infectées par VVhE7 produisent aussi une protéine cytoplasmique de 19-20 kDa spécifiquement reconnue par l'antisérum anti-E7 (fig. 7, bande 3, la bande 4 correspond à une immunoprécipitation avec un sérum non immun).

Pour E5, aucun antisérum n'étant disponible, l'intégration du bloc d'expression de E5 dans le virus de la vaccine est vérifiée par analyse de l'ADN selon la technique de Southern.

Exemple 6 : Effet de la vaccination par VVhE6 et VVhE7 sur le développement des tumeurs induites par HPV-16.

Des groupes de rats femelles (Fisher) agées de 4 semaines sont vaccinées par injection intradermale de 5.10$^7$ ufp (dans 100 $\mu$l) des différents virus recombinants. Ils sont soumis à une injection de rappel avec la même dose après 12 jours.

Entre le 16e et le 17e jour, ils sont éprouvés avec une lignée obtenue par co-transfection dans des cellules primaires de rat : (lignée RE01).

1) d'un plasmide comportant le génome de HPV-16 sous contrôle du LTR de rétrovirus de Moloney (22) ;

2) d'un plasmide codant pour un gène de résistance à G418 et pour EJ-RAS, qui ne transforme que des cellules de lignées établies ou exprimant un gène immortalisant.

2 x 10$^4$ cellules sont injectées dans un volume de 200 $\mu$l de tampon MEM.BME sans sérum par voie sous-cutanée.

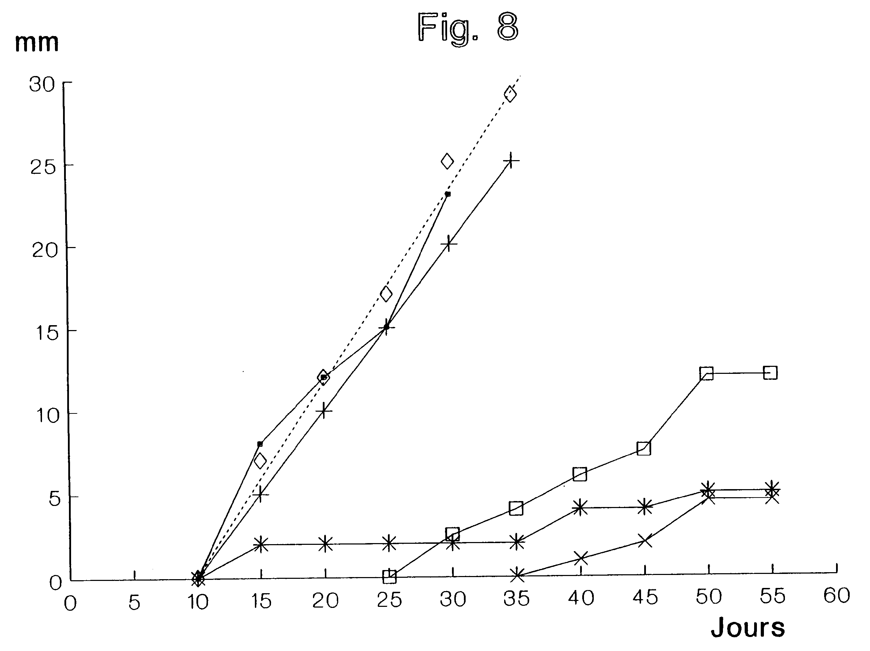

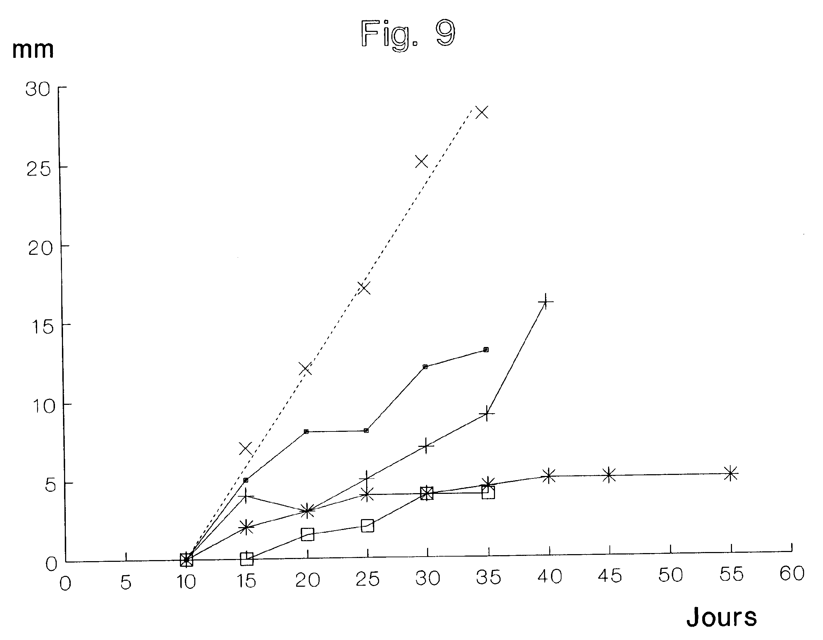

D'après les figures 8 et 9, on fait les constatations suivantes :

- les animaux témoins, non vaccinés, développent des tumeurs détectables 10 jours après l'inoculation des cellules transformées (voir lignes tiretées).
- les animaux vaccinés avec VVhE6 ne développent pas de tumeur dans 2 cas sur 7 ($\cong$ 100 jours). Lorsque des tumeurs apparaissent, dans 3 cas leur développement est ralenti de façon très significative et pour 2 animaux l'apparition des tumeurs est retardée (24 et 34 jours au lieu de 10 jours). Enfin, dans deux cas, le développement des tumeurs est identique à celui présenté par les animaux du lot témoin.
- les animaux vaccinés avec VVhE7 ne développent pas de tumeur dans 3 cas sur 7 ($\cong$ 100 jours). Dans les 4 autres cas, les tumeurs apparaissent dès le 10e jour, mais leur développement est significativement retardé.

Afin de tester les effets protecteurs de ces vaccinations, les animaux qui dans les deux lots n'ont pas développé de tumeurs sont repris et éprouvés 80 jours après la première épreuve d'inoculation avec 10 fois la dose initiale, c'est-à-dire 2 x 10$^5$ cellules. Dans de telles conditions d'inoculation, des animaux témoins développent des tumeurs après 4 jours.

- les animaux qui avaient été vaccinés par VVhE6 développent des tumeurs de façon identique aux animaux témoins.
- par contre, les animaux préalablement vaccinés avec VVhE7 même dans ces conditions ne développent pas de tumeurs ($\cong$ 100 jours).

Ces résultats ainsi que les précédents mettent en avant le rôle important que jouent les antigènes E7 dans la protection contre le développement des tumeurs induites par HPV-16.

Afin de vérifier les résultats ci-dessus deux autres séries d'expériences sont réalisées avec d'autres lignées cellulaires. Des groupes de rats femelles agées de 4 semaines sont vaccinées par injection intradermale des virus recombinants comme décrit précédemment. Les rats vaccinés par les virus recombinants sont éprouvés avec 2.10$^4$ ou 10$^5$ cellules primaires (lignée RE31) de rat préparées selon le protocole décrit précédemment. Le nombre d'animaux ayant rejeté les tumeurs ou chez lesquels on observe un retard dans l'apparition de celles-ci est donné dans la première partie du tableau II (Première épreuve). Dans ce tableau VV0 correspond à un virus de la vaccine recombinant témoin n'exprimant pas une protéine de HPV). Puis, des rats ayant rejeté une première fois les tumeurs sont à nouveau éprouvés mais en utilisant une concentration supérieure de cellules primaires ($2.10^5$ au lieu de $2.10^4$ ou $10^5$). Le résultats obtenus sont présentés dans la seconde partir du tableau II (Deuxième épreuve).

## TABLEAU II

| Virus recombinant | Rejet | Retard dans l'apparition de la tumeur | Total |
|---|---|---|---|
| Première épreuve par injection de $2x10^4$ cellules de la lignée RE31 par rat | | | |
| VVhE6 | 3/18 | 5/18 | 8/18 |
| VVhE7 | 3/17 | 5/17 | 9/17 |
| VV0 | 0/17 | 0/17 | 0/17 |
| Première épreuve par injection de $10^5$ cellules de la lignée RE31 par rat | | | |
| VVhE6 | 7/20 | 1/20 | 8/20 |
| VVhE7 | 5/20 | 0/20 | 5/20 |
| VV0 | 1/20 | 0/20 | 1/20 |
| Deuxième épreuve par injection de $2x10^5$ cellules de la lignée RE31 par rat | | | |
| VVhE6 | 2/4 | 0/4 | 2/4 |
| VVhE7 | 3/4 | 1/4 | 4/4 |
| VV0 | 0/5 | 0/5 | 0/5 |

Dans une autre série d'expériences, les lignées de cellules primaires de rats, servant à éprouver les rats vaccinés, sont co-transfectées par :

1) un plasmide comportant le génome de HPV-16 sous le contrôle de son propre promoteur,

2) un plasmide codant pour un gène de résistance à G418 et pour EJ-RAS, qui ne transforme que des cellules de lignées établies ou exprimant un gène immortalisant.

On obtient ainsi la lignée RE604. Le tableau III présente les résultats obtenus dans ces conditions expérimentales.

## TABLEAU III

Epreuve par injection de $2x10^4$ cellules de la lignée RE604 par rat

| Virus recombinant | Rejet | Retard dans l'apparition de la tumeur | Total |
|---|---|---|---|
| VVhE6 | 0/30 | 6/30 | 6/30 |
| VVhE7 | 7/40 | 10/40 | 17/40 |
| VVhE6/VVhE7 | 0/10 | 2/10 | 2/10 |
| VV0 | 0/30 | 0/30 | 0/30 |

Les résultats de ces expériences mettent en avant le rôle important que jouent les antigènes E7 et E6 dans la protection contre le dévelopement des tumeurs induites par HPV-16

Dans toutes les lignées cellulaires, l'expression de la protéine E7 est vérifiée par immunoprécipitation. L'action protectrice exercée par l'antigène E7 n'est donc pas dépendante de la lignée considérée.

Les souches suivantes ont été déposées le 24 février 1989 à la collection Nationale de Cultures de Microorganismes de l'Institut Pasteur (Paris) :

- E. coli pTG2198 sous le n° I-837.
- E. coli pTG2199 sous le n° I-838.
- E. coli pTG3180 sous le n° I-839.

**REFERENCES**

(1) PFISTER, H. (1987) in : The Papovaviridae : The papillomaviruses (édition, Salzman, N.P. et Howley, P.M.) Plenum Press, New York, p. 1-38.

(2) SCHLEGEL, R. et al. (1986). SCIENCE 233, 464-467.

(3) DANOS, O. et M. YANIV (1987). In. Cancer Cells 5 : Papillomaviruses, Cold Spring Harbor Laboratory, Cold Spring Harbor, N.Y.

(4) KANDA, T. et al. (1988). J. Virol. 62, 610-613.

(5) VOUSDEN, K. H. et al. (1988). Oncogene Res. 3, 1-9.

(6) BEDELL, M.A. et al. (1987). J. Virol. 61, 3635 3640.

(7) Zur HAUSEN, H. et SCHNEIDER, A. (1987). in : The Papovaviridae : The papillomaviruses (edition, Salzman, N.P. et Howley, P.M.) Plenum Press, New York, p. 245-263.

(8) WECK, P.K. et WHISNANT, J.K. (1987) in : The papillomaviruses (edition, Sulzman, N.P. et Howley, P.M.) Plenum Press, New York, p. 393-402.

(9) BINETRUY, B. et al. (1982). EMBO J. 82, 621-628.

(10) KIENY, M.P. et al. (1983). Gene 26, 91-99.

(11) KIENY, P.P. et al. (1984). Nature 312, 163-166.

(12) DAVIS G. et al. (1986). Basic methods in molecular biology (Elsevier).

(13) ANDROPHY, E.J. et al. (1987) in : The Papovaviridae : The papillomaviruses (edition, Salzman, N.P. et Howley, P.M.) Plenum Press, New York, p. 79-85.

(14) SCHLEGEL, R. et WADE-GLASS, M. (1987) in : The papillomaviruses (edition, Salzman, N.P. et Howley, P.M.) Plenum Press, New York, p 87-91.

(15) SCHILLER et al. (1984). PNAS 81, 7880.

(16) ANDROPHY, E.J. et al. (1986). Science 230, 442-445.

(17) BURCKHARDT, A. et al. (1987). EMBO J. 6, 2381-2385.

(18) GRISONI, M. et al. (1984). Virology 135, 406-416.

(19) LATHE, R. et al. (1987). GENE 57, 193-201.

(20) ANDROPHY, E.J. et al. (1987). EMBO J. 6, 989-992.

(21) SHOTKIN et WETTSTEIN (1986), PNAS 83, 1689-1694.

(22) STOREY, A. et al. (1988), EMBO J. 7, 1815-1820.

**Revendications**

**Revendications pour les Etats contractants suivants : AT, BE, CH, DE, DK, FR, GB, IT, LI, LU, NL, SE**

1. Une composition pharmaceutique destinée au traitement ou à la prévention d'une infection ou tumeur à papillomavirus qui comprend un poxvirus recombinant dans le génome duquel est inséré un fragment d'ADN hétérologue codant pour une protéine sélectionnée parmi les protéines $E_5$, $E_6$ et $E_7$ originaires d'un papillomavirus ou pour une région essentielle de ladite protéine ; ledit fragment d'ADN étant placé sous le contrôle des éléments nécessaires à son expression dans des cellules de mammifères.

2. Une composition pharmaceutique selon la revendication 1 qui comprend un poxvirus recombinant dans le génome duquel est inséré un fragment d'ADN hétérologue codant pour une protéine sélectionnée parmi les protéines $E_5$, $E_6$ et $E_7$ originaires d'un papillomavirus ; ledit fragment d'ADN étant placé sous le contrôle des éléments nécessaires à son expression dans des cellules de mammifères.

3. Une composition pharmaceutique selon la revendication 2 qui comprend un poxvirus recombinant dans le génome duquel est inséré un fragment d'ADN hétérologue codant pour une protéine sélectionnée parmi les protéines $E_5$, $E_6$ et $E_7$ originaires d'un papillomavirus de type HPV-16.

4. Une composition pharmaceutique selon l'une des revendications 2 ou 3 qui comprend un virus de la vaccine recombinant dans le génome duquel est inséré un fragment d'ADN hétérologue codant pour une protéine sélectionnée parmi les protéines $E_5$, $E_6$ et $E_7$ originaires d'un papillomavirus.

5. Une composition pharmaceutique selon l'une des revendications 2, 3 ou 4 qui comprend un poxvirus recombinant dans le génome duquel est inséré un fragment d'ADN hétérologue codant pour la protéine $E_5$ originaire d'un papillomavirus.

6. Un composition pharmaceutique selon l'une des revendications 2, 3 ou 4 qui comprend un poxvirus recombinant dans le génome duquel est inséré un fragment d'ADN hétérologue codant pour la protéine $E_6$ originaire d'un papillomavirus.

7. Une composition pharmaceutique selon l'une des revendications 2, 3 ou 4 qui comprend un poxvirus recombinant dans le génome duquel est inséré un fragment d'ADN hétérologue codant pour la protéine $E_7$ originaire d'un papillomavirus.

8. Une composition pharmaceutique selon l'une des revendications 1 à 7, caractérisée en ce que la position -3 de la région d'initiation de la traduction comporte un A ou un G.

9. Une composition pharmaceutique selon l'une des revendications 1 à 8, caractérisée en ce que le poxvirus est vivant ou tué.

10. Une composition pharmaceutique selon l'une des revendications 1 à 9, caractérisée en ce qu'elle comporte un support pharmaceutiquement acceptable permettant son administration par injection à l'homme ou à l'animal.

**Revendications pour l'Etat contractant suivant : ES**

1. Procédé de préparation d'une composition pharmaceutique destinée au traitement ou à la prévention d'une infection ou tumeur à papillomavirus qui comprend la formulation d'un poxvirus recombinant dans le génome duquel est inséré un fragment d'ADN hétérologue codant pour une protéine sélectionnée parmi les protéines $E_5$, $E_6$ et $E_7$ originaires d'un papillomavirus ou pour une région essentielle de la dite protéine ; le dit fragment d'ADN étant placé sous le contrôle des éléments nécessaires à son expression dans des cellules de mammifères, avec un support pharmaceutiquement acceptable.

2. Procédé selon la revendication 1 dans lequel le poxvirus est un poxvirus recombinant dans le génome duquel est inséré un fragment d'ADN hétérologue codant pour une protéine sélectionnée parmi les protéines $E_5$, $E_6$ et $E_7$ originaires d'un papillomavirus ; le dit fragment d'ADN étant placé sous le contrôle des éléments nécessaires à son expression dans des cellules de mammifères.

3. Procédé selon la revendication 2 dans lequel le poxvirus est un poxvirus recombinant dans le génome duquel est inséré un fragment d'ADN hétérologue codant pour une protéine sélectionnée parmi les protéines $E_5$, $E_6$ et $E_7$ originaires d'un papillomavirus de type HPV-16.

4. Procédé selon l'une des revendications 2 ou 3 dans lequel le poxvirus est un virus de la vaccine recombinant dans le génome duquel est inséré un fragment d'ADN hétérologue codant pour une protéine sélectionnée parmi les protéines $E_5$, $E_6$ et $E_7$ originaires d'un papillomavirus.

5. Procédé selon l'une des revendications 2, 3 ou 4 dans lequel le poxvirus est un poxvirus recombinant dans le génome duquel est inséré un fragment d'ADN hétérologue codant pour la protéine $E_5$ originaire d'un papillomavirus.

6. Procédé selon l'une des revendications 2, 3 ou 4 dans lequel le poxvirus est un poxvirus recombinant dans le génome duquel est inséré un fragment d'ADN hétérologue codant pour la protéine $E_6$ originaire d'un papillomavirus.

EP 0 462 187 B1

7. Procédé selon l'une des revendications 2, 3 ou 4 dans lequel le poxvirus est un poxvirus recombinant dans le génome duquel est inséré un fragment d'ADN hétérologue codant pour la protéine $E_7$ originaire d'un papillomavirus.

8. Procédé selon l'une des revendications 1 à 7, caractérisé en ce que la position -3 de la région d'initiation de la traduction comporte un A ou un G.

9. Procédé selon l'une des revendications 1 à 8, caractérisé en ce que le poxvirus est vivant ou tué.

10. Procédé selon l'une des revendications 1 à 9, caractérisé en ce que le support pharmaceutiquement acceptable permet son administration par injection à l'homme ou à l'animal.

**Patentansprüche**

**Patentansprüche für folgende Vertragsstaaten : AT, BE, CH, DE, DK, FR, GB, IT, LI, LU, NL, SE**

1. Pharmazeutische Zusammensetzung, bestimmt zur Behandlung oder Vorbeugung einer Infektion oder eines Tumors durch Papillomavirus, umfassend ein rekombinantes Poxvirus in dessen Genom ein heterologes DNA-Fragment eingeführt ist, das kodiert für ein Protein, ausgewählt unter den ursprünglichen Proteinen $E_5$, $E_6$ und $E_7$ eines Papillomavirus oder für eine essentielle Region dieses Proteins; wobei dieses DNA-Fragment unter die Kontrolle von für seine Expression in Säugetier-Zellen notwendige Elemente plaziert ist.

2. Pharmazeutische Zusammensetzung nach Anspruch 1, umfassend ein rekombinantes Poxvirus, in dessen Genom ein heterologes DNA-Fragment eingeführt ist, das für ein Protein kodiert, das ausgewählt ist unter den ursprünglichen Proteinen $E_5$, $E_6$ und $E_7$ eines Papillomavirus; wobei dieses DNA-Fragment unter die Kontrolle von für seine Expression in Säugetier-Zellen notwendige Elemente plaziert ist.

3. Pharmazeutische Zusammensetzung nach Anspruch 2, umfassend ein rekombinantes Poxvirus, in dessen Genom ein heterologes DNA-Fragment eingeführt ist, das für ein Protein kodiert, das ausgewählt ist unter den ursprünglichen Proteinen $E_5$, $E_6$ und $E_7$ eines Papillomavirus vom Typ HPV-16.

4. Pharmazeutische Zusammensetzung nach einem der Ansprüche 2 oder 3, umfassend ein rekombinantes Kuhpockenvirus, in dessen Genom ein heterologes DNA-Fragment eingeführt ist, das für ein Protein kodiert, das ausgewählt ist unter den ursprünglichen Proteinen $E_5$, $E_6$ und $E_7$ eines Papillomavirus.

5. Pharmazeutische Zusammensetzung nach einem der Ansprüche 2, 3 oder 4, umfassend ein rekombinantes Poxvirus, in dessen Genom ein heterologes DNA-Fragment eingeführt ist, das für das ursprüngliche Protein $E_5$ eines Papillomavirus kodiert.

6. Pharmazeutische Zusammensetzung nach einem der Ansprüche 2, 3 oder 4, umfassend ein rekombinantes Poxvirus, in dessen Genom ein heterologes DNA-Fragment eingeführt ist, das für das ursprüngliche Protein $E_6$ eines Papillomavirus kodiert.

7. Pharmazeutische Zusammensetzung nach einem der Ansprüche 2, 3 oder 4, umfassend ein rekombinantes Poxvirus, in dessen Genom ein heterologes DNA-Fragment eingeführt ist, das für ein ursprüngliches Protein $E_7$ eines Papillomavirus kodiert.

8. Pharmazeutische Zusammensetzung nach einem der Ansprüche 1 bis 7, dadurch gekennzeichnet, daß die 3-Stellung der Initiierungs-Region der Translation ein A oder ein G umfaßt.

9. Pharmazeutische Zusammensetzung nach einem der Ansprüche 1 bis 8, dadurch gekennzeichnet, daß das Poxvirus lebend oder abgetötet ist.

10. Pharmazeutische Zusammensetzung nach einem der Ansprüche 1 bis 9, dadurch gekennzeichnet, daß sie einen brauchbaren pharmazeutischen Träger umfaßt, der ihre Verabreichung durch Injektion an Menschen oder Tiere ermöglicht.

13

EP 0 462 187 B1

**Patentansprüche für folgenden Vertragsstaat : ES**

1. Verfahren zur Herstellung einer pharmazeutischen Zusammensetzung, bestimmt zur Behandlung oder Vorbeugung einer Infektion oder eines Tumors durch Papillomavirus, umfassend die Formulierung eines rekombinanten Poxvirus, in dessen Genom ein heterologes DNA-Fragment eingeführt ist, das kodiert für ein Protein, ausgewählt unter den ursprünglichen Proteinen $E_5$, $E_6$ und $E_7$ eines Papillomavirus, oder für eine essentielle Region dieses Proteins; wobei dieses DNA-Fragment unter die Kontrolle von die für seine Expression in Säugetier-Zellen notwendige Elemente plaziert ist, mit einem pharmazeutisch brauchbaren Träger.

2. Verfahren nach Anspruch 1, bei dem das Poxvirus ein rekombinantes Poxvirus ist, in dessen Genom ein heterologes DNA-Fragment eingeführt ist, das kodiert für ein Protein, das ausgewählt ist unter den ursprünglichen Proteinen $E_5$, $E_6$ und $E_7$ eines Papillomavirus; wobei dieses DNA-Fragment unter die Kontrolle von für seine Expression in Säugetier-Zellen notwendige Elemente plaziert ist.

3. Verfahren nach Anspruch 2, worin das Poxvirus ein rekombinantes Poxvirus ist, in dessen Genom ein heterologes DNA-Fragment eingeführt ist, das kodiert ist für ein Protein, das ausgewählt ist aus den ursprünglichen Proteinen $E_5$, $E_6$ und $E_7$ eines Papillomavirus vom Typ HPV-16.

4. Verfahren nach einem der Ansprüche 2 oder 3, worin das Poxvirus ein rekombinantes Kuhpockenvirus ist, in dessen Genom ein heterologes DNA-Fragment eingeführt ist, das kodiert für ein Protein, das ausgewählt ist aus den ursprünglichen Proteinen $E_5$, $E_6$ und $E_7$ eines Papillomavirus.

5. Verfahren nach einem der Ansprüche 2, 3 oder 4, worin das Poxvirus ein rekombinantes Poxvirus ist, in dessen Genom ein heterologes DNA-Fragment eingeführt ist, das kodiert für das ursprüngliche Protein $E_5$ eines Papillomavirus.

6. Verfahren nach einem der Ansprüche 2, 3 oder 4, worin das Poxvirus ein rekombinantes Poxvirus ist, in dessen Genom ein heterologes DNA-Fragment eingeführt ist, das kodiert für das ursprüngliche Protein $E_6$ eines Papillomavirus.

7. Verfahren nach einem der Ansprüche 2, 3 oder 4, worin das Poxvirus ein rekombinantes Poxvirus ist, in dessen Genom ein heterologes DNA-Fragment eingeführt ist, das kodiert für das ursprüngliche Protein $E_7$ eines Papillomavirus.

8. Verfahren nach einem der Ansprüche 1 bis 7, dadurch gekennzeichnet, daß die 3-Stellung der Initiierungs-Region der Translation ein A oder ein G umfaßt.

9. Verfahren nach einem der Ansprüche 1 bis 8, dadurch gekennzeichnet, daß das Poxvirus lebend oder abgetötet ist.

10. Verfahren nach einem der Ansprüche 1 bis 9, dadurch gekennzeichnet, daß der pharmazeutisch brauchbare Träger die Verabreichung durch Injektion an Menschen oder Tiere ermöglicht.


**Claims**

**Claims for the following Contracting States : AT, BE, CH, DE, DK, FR, GB, IT, LI, LU, NL, SE**

1. Pharmaceutical preparation intended for the treatment or prevention of a papillomavirus infection or tumour, which preparation comprises a recombinant pox virus, into the genome of which is inserted a fragment of heterologous DNA coding for a protein selected from the proteins $E_5$, $E_6$ and $E_7$ originating from a papillomavirus or for an essential section of the said protein; the said DNA fragment being placed under the control of the factors necessary for its expression in mammalian cells.

2. Pharmaceutical preparation according to claim 1 comprising a recombinant pox virus, into the genome of which is inserted a fragment of heterologous DNA coding for a protein selected from the proteins $E_5$, $E_6$ and $E_7$ originating from a papillomavirus; the said DNA fragment being placed under the control of the factors necessary for its expression in mammalian cells.

14

3. Pharmaceutical preparation according to claim 2 comprising a recombinant pox virus, into the genome of which is inserted a fragment of heterologous DNA coding for a protein selected from the proteins $E_5$, $E_6$ and $E_7$ originating from a type HPV-16 papillomavirus.

4. Pharmaceutical preparation according to one of claims 2 or 3 comprising a recombinant vaccinia virus, into the genome of which is inserted a fragment of heterologous DNA coding for a protein selected from the proteins $E_5$, $E_6$ and $E_7$ originating from a papillomavirus.

5. Pharmaceutical preparation according to one of claims 2, 3 or 4 comprising a recombinant pox virus, into the genome of which is inserted a fragment of heterologous DNA coding for protein $E_5$ originating from a papillomavirus.

6. Pharmaceutical preparation according to one of claims 2, 3 or 4 comprising a recombinant pox virus, into the genome of which is inserted a fragment of heterologous DNA coding for protein $E_6$ originating from a papillomavirus.

7. Pharmaceutical preparation according to one of claims 2, 3 or 4 comprising a recombinant pox virus, into the genome of which is inserted a fragment of heterologous DNA coding for protein $E_7$ originating from a papillomavirus.

8. Pharmaceutical preparation according to one of claims 1 to 7, characterised in that position -3 of the translation initiation section comprises an A or a G.

9. Pharmaceutical preparation according to one of claims 1 to 8, characterised in that the pox virus is living or killed.

10. Pharmaceutical preparation according to one of claims 1 to 9, characterised in that it comprises an acceptable pharmaceutical carrier allowing its administration by injection into man or animals.

**Claims for the following Contracting State : ES**

1. Process for the production of a pharmaceutical preparation intended for the treatment or prevention of a papillomavirus infection or tumour, which preparation comprises the formulation of a recombinant pox virus, into the genome of which is inserted a fragment of heterologous DNA coding for a protein selected from the proteins $E_5$, $E_6$ and $E_7$ originating from a papillomavirus or for an essential section of the said protein; the said DNA fragment being placed under the control of the factors necessary for its expression in mammalian cells, with an acceptable pharmaceutical carrier.

2. Process according to claim 1 in which the pox virus is a recombinant pox virus, into the genome of which is inserted a fragment of heterologous DNA coding for a protein selected from the proteins $E_5$, $E_6$ and $E_7$ originating from a papillomavirus; the said DNA fragment being placed under the control of the factors necessary for its expression in mammalian cells.

3. Process according to claim 2 in which the pox virus is a recombinant pox virus, into the genome of which is inserted a fragment of heterologous DNA coding for a protein selected from proteins $E_5$, $E_6$ and $E_7$ originating from a type HPV-16 papillomavirus.

4. Process according to one of claims 2 or 3 in which the pox virus is a recombinant vaccinia virus, into the genome of which is inserted a fragment of heterologous DNA coding for a protein selected from the proteins $E_5$, $E_6$ and $E_7$ originating from a papillomavirus.

5. Process according to one of claims 2, 3 or 4 in which the pox virus is a recombinant pox virus, into the genome of which is inserted a fragment of heterologous DNA coding for protein $E_5$ originating from a papillomavirus.

6. Process according to one of claims 2, 3 or 4 in which the pox virus is a recombinant pox virus, into the genome of which is inserted a fragment of heterologous DNA coding for protein $E_6$ originating from a papillomavirus.

7. Process according to one of claims 2, 3 or 4 in which the pox virus is a recombinant pox virus, into the genome of which is inserted a fragment of heterologous DNA coding for protein $E_7$ originating from a papillomavirus.

8. Process according to one of claims 1 to 7, characterised in that position -3 of the translation initiation section comprises an A or a G.

9. Process according to one of claims 1 to 8, characterised in that the pox virus is living or killed.

10. Process according to one of claims 1 to 9, characterised in that the acceptable pharmaceutical carrier allows its administration by injection into man or animals.

# FIG. 1

# F I G. 2

# FIG. 3

# Fig. 4

M13TG130

EcoRI

E7

E6

PstI   SphI   PstI

1772 pb

1316 pb

# Fig. 5

# FIG. 6

ATGACAAATCT TGATACTGCATCCACAACAT TACTGGCGTGCT T T T TGCT

1                                                                      50

T TGCT T T TGTGTGCT T T TGTGTGTCTGCCTAT TAATACGTCCGCTGCT T T

                                                                       100

TGTCTGTGTCTACATACACATCAT TAATACTAT TGGTAT TACTAT TGTGG

                                                                       150

ATAACAGCAGCCTCTGCGT T TAGGTGT T T TAT TGTATATAT TGTAT T TGT

                                                                       200

T TATATACCAT TAT T T T TAATACATACACATGCACGCT T T T TAAT TACATAA

                                                                       250

# FIG. 7

Fig. 8

Fig. 9